# EUROPEAN PATENT APPLICATION

(11) **EP 1 566 439 A1**
(43) Date of publication of application: **24.08.2005**
(21) Application number: 04003853.1
(22) Date of filing: 20.02.2004
(51) Int. Cl.: C12N 15/12, C07K 14/705, A61K 48/00, A61K 39/00, A61K 31/35, A61K 31/05, A61K 31/66, A61P 3/04, G01N 33/50, C12Q 1/68

(54) **Preventing or treating obesity**

(71) Applicant: NESTEC S.A., 1800 Vevey (CH)
(72) Inventor: The designation of the inventor has not yet been filed
(74) Representative: Chautard, Cécile

(57) **Abstract**

The present invention pertains to a method for preventing and/or treating obesity and associated disorders. In particular, the present invention relates to substances and/or compositions stimulating and/or modifying endogenous CD_{1d} function. According to another aspect the present invention also provides a method for screening for compounds suitable for use in the method and the composition of the present invention.

## Description

The present invention pertains to a method for preventing and/or treating obesity. Specifically, the present invention relates to substances and/or compositions that modify, in particular simulate endogenous CD_{1d} function. According to another aspect the present invention also provides a method for screening for compounds suitable for use in the method and the composition of the present invention.

In animals, specifically mammals, the biological role of body fat generally resides in representing a source of energy for the individual for sustaining times of decreased nutritional supply. The regulation of body fat is controlled in part by appetite but also by energy expenditure. An important component of said energy expenditure is non-shivering thermogenesis, occurring mainly in brown adipose tissue via the so called uncoupling protein, a proton channel located exclusively in the inner mitochondrial membrane. By allowing protons to equilibrate across the inner mitochondrial membrane, this protein uncouples oxidative phosphorylation from ATP production and thus converts stored energy into heat rather than work.

Also, in humans, body weight homeostasis is considered to involve controlled thermogenesis. However, since normally only low levels of brown adipose tissue is present, a large portion of thermogenesis is deemed to be mediated by muscle and white adipose tissue.

In the recent past body weight disorders have emerged to represent major health problems in industrialized countries. Examples for such disorders are anorexia nervosa and bulimia nervosa which together affect about 0.2% of the female population of the western world. Obesity, the most prevalent disorder afflicts from about 30% to 50% of middle-aged people.

Obesity is generically defined as an excess of body fat relative to lean body mass and may contribute to a number of other diseases, such as e.g. sleep apnoea, hernias, flat feet, arthritis, osteoarthritis, some type of cancers, varicose veins, gout, hypertension, respiratory problems, gall bladder disease and liver disease, coronary artery disease, atherosclerosis, stroke, and particularly diabetes.

The mechanisms that are involved in obesity-diabetes syndromes are presently not fully understood. The phenomenon of insulin resistance seems to be implicated, which is defined as a biological response to a given dose of insulin lower than expected. This phenomenon has been shown to be in part due to a down-regulation of the insulin receptor during the initial phases and a post-receptor abnormality which develops during the course of insulin resistance. Also, free fatty acids have been considered to be implicated, since the levels thereof are typically elevated in obesity, and fatty acids have been shown to affect insulin sensitivity.

Though obesity is occasionally thought to be a mere behavioral issue, the differential body composition observed between obese and normal subjects has been shown to result from differences in both metabolism and neurologic/metabolic interactions. These differences obviously seem to be based on a differential expression of a variety of genes.

At present, the nature of the genetic factors which control body composition is still for the most part unknown. The epidemiology of obesity implies that the disorders may exhibit inherited characteristics with obesity being controlled by a number of genetic loci. In this respect animals having mutations eventually leading to syndromes including obesity symptoms have been identified. Representative examples are mice containing the autosomal recessive mutations ob/ob (obese) and db/db (diabetes). These mutations have been found to be located on chromosomes 6 and 4, respectively, leading to clinically similar pictures of obesity, including hyperphagia, abnormalities in glucose and insulin metabolism, poor thermoregulation and non-shivering thermogenesis. The ob mutation resides in the gene encoding leptin, a hormone secreted by adipocytes and the db mutation resides in the leptin receptor gene. It has been found that the application of a diet to restore a regular ratio of body fat to lean body mass does not result in a normal habitus.

Other animals, exhibiting an obese phenotype include the yellow mutation at the agouti locus, which causes a pleiotropic syndrome that results in a moderate adult onset obesity, a yellow coat colour, a high incidence of tumour formation, and an abnormal anatomic distribution of body fat. Additionally, mutations at the fat and tubby loci cause moderately severe, maturity-onset obesity with somewhat milder abnormalities in glucose homeostasis than are observed in ob and db mice. Interleukin-6 (IL-6) deficient mice develop mature onset obesity and exhibit disturbed carbohydrate and lipid metabolism, and increased leptin levels. The immune-modulating cytokine IL-6 is expressed both in adipose tissue and centrally in hypothalamic nuclei that regulate body composition. Transgenic mice over-expressing the enzyme 11β hydroxysteroid dehydrogenase type 1 (11β HSD-1) in adipose tissue, which produces glucocorticoids from inactive 11-keto forms, develop visceral obesity that is exaggerated by a high-fat diet. Further, autosomal dominant mutations at the adipose locus of chromosome 7, have been shown to cause obesity. Other animals include fa/fa (fatty) rats, that are sensitive to cold, while their capacity for non-shivering thermogenesis being normal. Torpor seems to play a major role in the maintenance of obesity in these fa/fa rats.

Additional genes which are presently deemed to regulate obesity include hormones and their respective receptors-glucocorticoid, growth hormone, insulin-like growth factor (IGF-I), thyroid hormone, adiponectin, resistin, melanin-concentrating hormone (MCH), α-melanocyte stimulating hormone (α-MSH); neuropeptides and their respective receptors- including calcitonin-gene related peptide (CGRP), neuropeptide (NPY), β-3-adrenergic receptor; transcription factors including PPARs, RARs, HNFs, SREBPs; adipokines including TNF-α; eicosanoids, specifically prostaglandin E₂ (PGE₂) and prostacyclin (PGI₂); and enzymes including HMH CoA reductase and plasminogen activator inhibitor-1 (PAI-1).

In the art there have been many attempts to treat obesity. As a general means diets have been proposed, so as to provide a low calorie content only, but still providing to the individual the necessary proteins, vitamins and minerals. Most of these preparations are in the form of powders which are stirred up in water before intake. Yet, these preparations suffer from the inherent deficiencies of having an unpleasant tang which even persists as an after-taste a long time after the preparation has been ingested. In addition, according to the data obtained with ob/ob mice these preparations do not seem to be apt for treating obesity, since they cannot actually influence the underlying genetic disorders.

Also, the murine or human ob proteins may be administered to individuals suffering from defects or mutations in their corresponding obese (ob) gene. Consequently, these proteins may be utilized as a hormone-like substance to control, prevent or treat obesity and its related diseases and conditions. Yet, this type of medicament may only be applied in case the ob-gene is the cause of obesity in the respective individual. Also, the observation that most obese individuals have elevated serum levels of OB has prompted speculation that obesity is due to a decreased sensitvity to OB, rather than to a deficiency of OB. Thus, in terms of OB-based therapy, successful treatment of obesity may not arise until the molecular determinants of reduced OB responsiveness have been identified.

In view of the importance of understanding body weight homeostasis and the severity and prevalence of disorders, including obesity, all of which affect body weight and associated diseases, there exists a great need for providing a means of preventing and/or treating obesity and the disorders associated therewith.

Consequently, an object of the present invention is to provide a means for preventing and/or treating obesity.

This problem has been solved by providing a substance that is capable to essentially modify, in particular stimulate the endogenous CD_{1d} function in cells of the body.

In the figures,
Fig. 1 shows that CD_{1d} knockout mice fed on a regular diet, regardless of age and sex are heavier than their wild-type controls, i.e. obese;
Fig. 2 shows 8 month old CD_{1d} knockout mice exhibiting an obese phenotype compared to wild-type controls.
Fig. 3 shows differences in the weight of fat pads dissected from 8 month old CD1d knockout and wild-type controls.

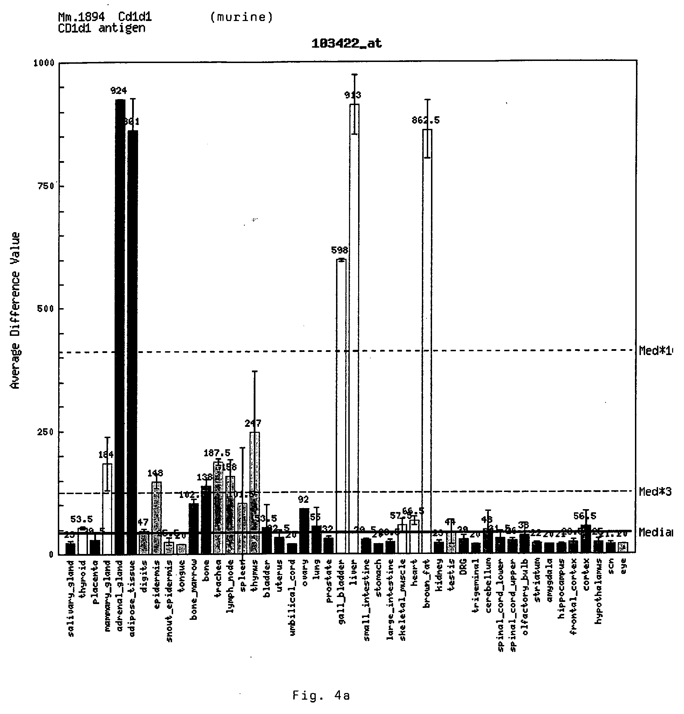

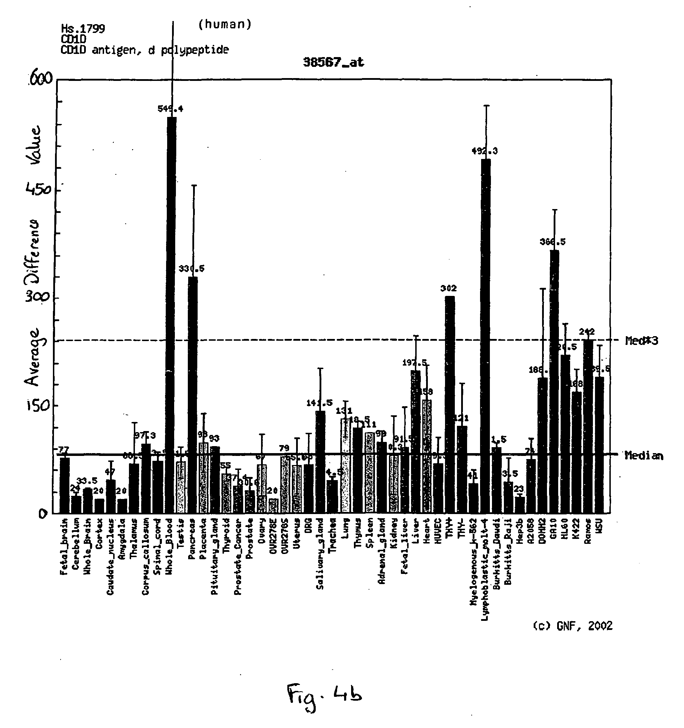

Figs. 4a and b show graphs indicating the approximate amount of CD_{1d} in different tissues in mice and human; high levels of CD_{1d} expression are observed in tissues important in regulating obesity including adipose tissue.

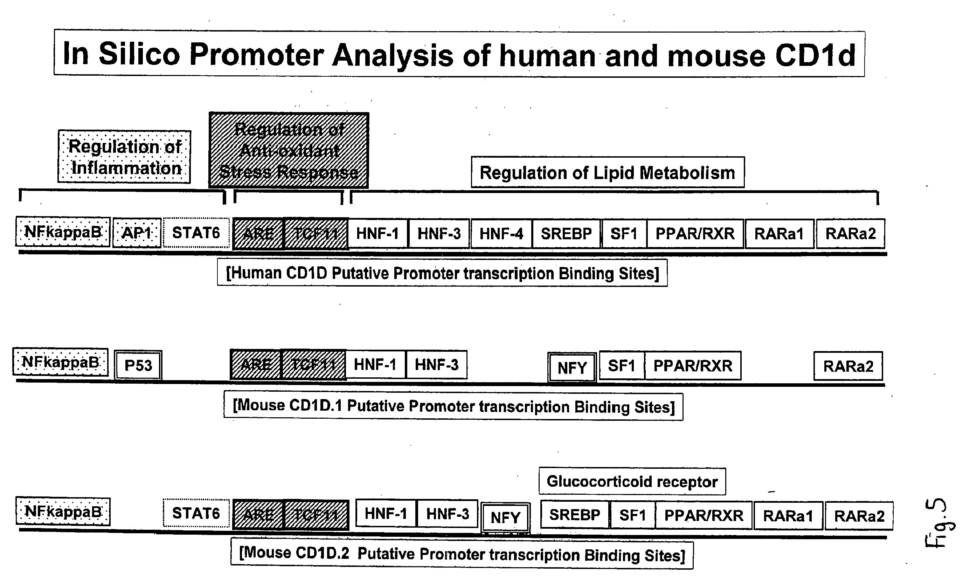

Fig. 5 illustrates, based on in silico analysis, that the human and mouse CD_{1d} promotors are capable of being regulated by a number of transcription factors known to play a key role in regulating lipid metabolism and obesity.

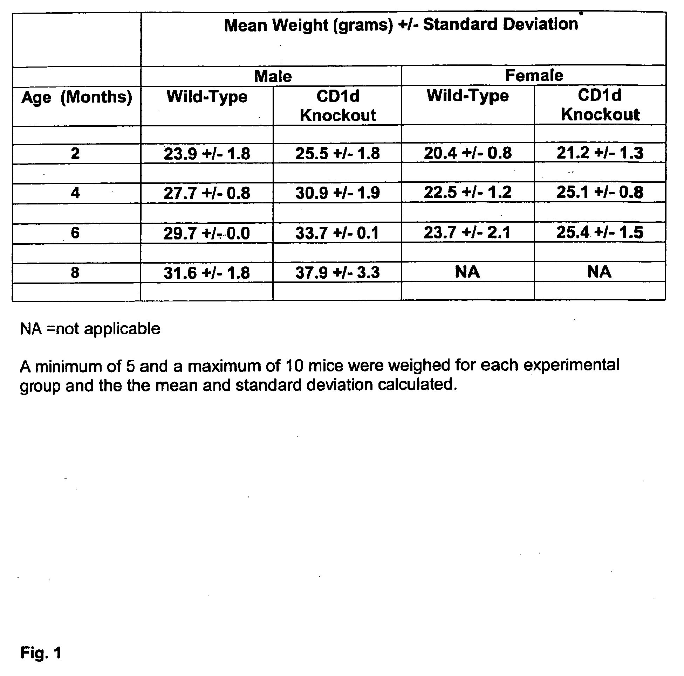

The present invention is essentially based on the finding that CD_{1d} knock out mice exhibit an obese phenotype as compared to wild type mice (cf. Figs. 1, 2 and 3). In addition, in these mice a deregulation of genes known to be implicated in lipid metabolism, in particular obesity and the associated disease diabetes mellitus could be observed.

CD_{1d} as such is a type 1 transmembrane MHC class 1 like protein that non-covalently associates with β₂-microglobulin. The CD_{1d} molecule is recognized by a T-cell receptor of natural killer T-cells (NKT) which play a role in immune modulatory and effectory reactions. It has been demonstrated that CD_{1d} may present lipids to NKT cells for their activation, which notion is supported by the CD_{1d} crystal structure having two highly hydrophobic grooves, necessary for presenting hydrophobic molecules such as lipids to the immune system.

Based on the findings according the present invention, CD_{1d} appears to be one of the key molecules, which when down regulated results in the symptoms of obesity and related disorders. Consequently, when modifying, in particular stimulating endogenous CD_{1d} function, those genes and/or transcripts involved in eliciting the recognized symptoms of obesity will be up or down-regulated, respectively, such that the level thereof is adapted to standard, i.e. as essentially prevailing in a non-obese individual.

The substance capable of modifying and/or stimulating the CD_{1d} transmembrane molecule's activity may be any substance interfering with the endogenous biological function of CD_{1d}, and in particular increasing association of CD_{1d} with endogenous or exogenous lipids. The substances are obtainable by a process comprising the steps of (a) contacting adipocytes and/or pre-adipocytes derived from white or brown adipocyte tissue with a substance of interest, (b) determining the effect of said substance on said adipocyte/pre-adipocyte cell development by screening for one or more of the following assays: (i) adipocyte hyperplasia (H&E), (ii) adipocyte hypertrophy (H&E) (iii) adipocyte apoptosis, (iv) adipocyte differentiation, (v) adipocyte dedifferentiation, (vi) adipocyte lipogenesis, (vii) production of adipokines (e.g. TNF-α, IL-6), (viii) production of anti-inflammatory molecules (e.g. IL-1 receptor antagonist), (ix) lipid uptake/transport, (x) adipocyte lipolysis, (xi) transcription factor activity e.g. PPAR, SREBP, RAR, HNF (xii) adipocyte endocrine functions (e.g. production of leptin, adiponectin, resistin) (xiii) ABC transporter activity e.g. ABC1, ABCD2, PMP70 (xiv) enzyme activity including lipases (e.g. secretory and cytosolic phospholipase A₂, lipoprotein lipase, lysosomal acid lipase, HMG CoA reductase) and secretion (e.g. plasminogen activator inhibitor-1) (xv) Cox-2 activity (xvi) lipoprotein receptor activity/expression, (d) comparing the results obtained with a control.

Such a control may e.g. be an assay, wherein cells of the same type have not been exposed to the test substance of interest (but otherwise treated/cultured in the same manner), wherein a decrease/inhibition of development indicators as determined in step b) is indicative for a substance capable of modifying and/or stimulating endogenous CD_{1d} function. Likewise another control may be cells lacking CD_{1d} that have been exposed to the test substance (negative control) or including a substance with a known positive effect on adipocyte/pre-adipocyte development (i.e. reducing or preventing adipocyte/pre-adipocyte development) in the assay and determining the difference in effect achieved by the substance investigated and the known substance (positive control).

Alternatively, the substances are also obtainable by a process comprising the steps above including the assays, with the proviso that after contacting adipocytes/pre-adipocytes derived from white or brown adipocyte tissue with a substance of interest, the same cells are then exposed to a substance or a mixture of substances known to increase adipose tissue size, especially a substance which induces/increases pre-adipocyte proliferation, differentiation and/or induces/increases adipocyte volume; e.g. glucocorticoids, insulin-like growth factor (IGF-I), angiotensin II, lipoproteins (VLDL, LDL, HDL), E2Fs, norepinephrine, fatty acids, prostacyclin, macrophage colony stimulating factor. It will be appreciated that the order of these two steps may be exchanged, i.e. first the cells are exposed to a substance known to increase adipose tissue size and in a next step a substance of interest is added.

A substance is considered to be active in the context of this application, in case it decreases/inhibits normal adipocyte development or any effect associated with increased adipose tissue mass, such as increased adipose proliferation, differentiation and/cell volume.

In principle, it will be appreciated that CD_{1d} activity may be stimulated and/or modified by substances acting on the genetic level or at the protein level.

Substances acting on the genetic level are compounds influencing, in particular stimulating transcription or translation of the CD_{1d} gene,- or restoring a gene defect by introducing polynucleotides sense to the CD_{1d} gene or the CD_{1d}-mRNA. Here, the essentially full transcript may be administered by means known of the art, such as liposome techniques or vectors (viral). According to a preferred embodiment such a substance may be an cDNA.

The terms oligonucleotide and polynucleotide, which are used herein interchangeably, include linear oligomers/polymers of natural or modified monomers or linkages, including desoxyribonucleosides, ribonucleosides, α-anomeric forms thereof, polyamide nucleic acids, and the like, capable of specifically binding to the target nucleic acid by way of a regular pattern of monomer-to-monomer interactions, such as Watson-Crick type of base pairing, Hoogsteen or reverse Hoogsteen types of base pairing, or the like. Usually the monomers are linked by phosphodiester bonds or analogs thereof to form oligonucleotides ranging in size from a few monomeric units, e.g. 3-5, to several 100 or even thousands of monomeric units.

The oligo-/polynucleotides may also contain pendent groups or moieties, to enhance specificity, nuclease resistance, delivery, or other property related to efficacy, such as e.g. cholesterol moieties, duplex intercalators such as acridine, poly-L-lysine, "end capping" with one or more nuclease-resistant linkage groups such as phosphorothioate, and the like. The corresponding oligonucleotide may be used for increasing transcription, RNA processing and/or translation of the CD_{1d}-mRNA, Consequently, the oligonucleotide may comprise exon, but also intron sequences of the CD_{1d} /target gene, as desired.

The nucleotide sequence of the human CD_{1d} gene or mRNA is obtainable from NCBI (Accession numbers: X14974 and NM_001766, respectively). Based on his general knowledge, the skilled person may select at least a portion of the non-coding region of the CD_{1d} gene, i.e. the promotor or operator region, respectively, and design an appropriate polynucleotide, that stimulates/increases transcription and/or translation of the CD_{1d} gene. Likewise, also the coding region of the CD_{1d} gene may serve as an agent for stimulating transcription or increasing the number of transcripts, respectively, of the CD_{1d} gene. Here, in particular the essentially full transcript may be administered by means known n the art, such as liposome techniques or (viral) vectors. According to a preferred embodiment such a substance may be an DNA or a cRNA (RNA-interference).

Yet, apart from the CD_{1d} gene being the target, also the activity of a number of regulatory molecules which control cellular homeostasis such as ceramides and/or glucosylceramides, may be modified such, that they exert the desired effect on the CD_{1d} molecule. To this end, the number of the glucosylceramide synthase transcripts may be increased by designing an polynucleotide sense to the coding part of the glucosylceramide synthase gene or glucosylceramide synthase mRNA. The nucleotide sequence of the glucosylceramide synthase gene is disclosed in Ichikawa et al., PNAS 93 (1996), 4638-4643, which document is incorporated herein by way of reference. Likewise, non coding regions may serve as a template for the objective polynucleotides, such as the polynucleotides binding to the promotor and operator regions, respectively. According to a preferred embodiment such a substance may be a DNA or a cRNA (RNA-interference).

Apart from raising the proliferation signal in the cells (cf. above) also the number of transcripts may be increased, which stimulate CD_{1d} activity in a different manner, such as e.g. the sphingomyelinase or ceramide synthase gene and/or the sphingomyelinase or ceramide synthase mRNA. In increasing the number of transcripts thereof, the same effect, i.e. a stimulation of CD_{1d} activity may be observed.

Apart from the genetic level, the biological activity of the CD_{1d} molecule itself may also be modified, in particular stimulated at the protein level, specifically by any substance binding to the CD_{1d} receptor on or in adipocytes cells and stimulating the endogenous biological functionality thereof.

According to a preferred embodiment the substance capable of modifying, in particular stimulating biological CD_{1d} function is a polypeptide or a peptide, in particular hydrophobic peptides, more preferably an antibody, or a part thereof, that binds to the CD_{1d} receptor and stimulates its biological function, such as blocking pre-adipocyte to adipocyte differentiation or stimulating adipocyte de-differentiation. As parts thereof, in particular mini-antibodies are envisaged lacking the F_{c}-part.

According to a preferred embodiment the substance capable of stimulating and/or modifying biological CD_{1d} function is a lipid derived from a plant, microbe or animal, including a phospholipid, ganglioside, sphingolipid, glycosphingolipid, phosphatidylinositol phosphate, sterol, glycerol, glyceride or fatty acid. These lipids may influence CD_{1d} function by directly binding the CD_{1d} molecule or indirectly by influencing CD_{1d} gene expression.

According to a preferred embodiment the substance capable of stimulating and/or modifying biological CD_{1d} function is a phenol, polyphenol or a molecule derived from Gingko . These molecules may influence CD_{1d} function by directly binding the CD_{1d} molecule or indirectly by influencing CD_{1d} gene expression.

According to an alternative embodiment the substance capable of stimulating and/or modifying biological CD_{1d} function is an organic compound obtained by chemical synthesis.

In principle, the substances of the present invention may also influence and stimulate the bidirectional trafficking of CD_{1d} to and from the membrane.

The substances may be included in any composition suitable for administering the substance to an individual, in particular a food composition, a cosmetic composition or a pharmaceutical composition.

The pharmaceutical compositions containing at least one of the substances according to the invention capable of stimulating or modifying the CD_{1d} surface molecule's activity can be administered for prophylactic and/or therapeutic treatments. In therapeutic applications, compositions are administered to a patient already suffering from obesity or associated complications, in an amount sufficient to cure or at least partially arrest the symptoms of the disease. An amount adequate to accomplish this is defined as "a therapeutically effective dose". Amounts effective for this will depend on the severity of the disease and the weight and general state of the patient.

In prophylactic applications, compositions containing at least one of the substances according to the invention capable of stimulating or modifying the CD_{1d} surface molecule's activity are administered to a patient susceptible to or otherwise at risk of obesity or associated conditions. Such an amount is defined to be "a prophylactic effective dose". In this use, the precise amounts again depend on the patient's state of health and weight.

The compounds of the invention are preferably administered with a pharmaceutical acceptable carrier, the nature of the carrier differing with the mode of administration, for example parenteral, intravenous, oral and topical (including ophthalmic) routes.

The desired formulations may be made using a variety of different excipients including, for example, pharmaceutical grades of mannitol, lactose, starch, magnesium stearate, sodium saccharin cellulose, magnesium carbonate. This composition may be a tablet, a capsule, a pill, a solution, a suspension, a syrup, a dried oral supplement, a wet oral supplement, dry tube-feeding, wet tube-feeding etc.. In order to control the drug release, sustained-release formulations can also be used.

The kind of the carrier/excipient and the amount thereof will depend on the nature of the substance and the mode of drug delivery and/or administration contemplated. E.g., for formulations containing weakly soluble sense oligonucleotides, micro-emulsions may be employed, for example by using a non-ionic surfactant such as Tween 80 in an amount of about 0.04-0.05% (w/v), to increase solubility. Other components may include antioxidants, such as ascorbic acid, hydrophilic polymers, such as, monosaccharides, disaccharides, and other carbohydrates including cellulose or its derivatives, dextrins, chelating agents, such as EDTA, and like components well known to those in the pharmaceutical sciences. These various components utilized provide a variety of functions, including regulation of drug concentration, regulation of solubility, chemical stabilization, regulation of viscosity, absorption enhancement, regulation of pH, and the like. For example, in water soluble formulations the pharmaceutical composition preferably includes a buffer such as a phosphate buffer, or other organic acid salts, preferably at a pH of between about 7 and 8.

It will be appreciated that the skilled person will, based on his own knowledge, select the appropriate components and galenic form to target the active compound to the tissue of interest, e.g. adipose tissue, colon, stomach, pancreas, gall bladder, skin, kidney or liver, taking into account the route of administration which may be by way of injection, topical application, intranasal administration, administration by implanted or transdermal sustained release systems, and the like.

The objective substance may also be formulated in a cosmetic product, such as lotions, creams, or ointments. This proves specifically advantageous for essentially stimulating CD_{1d} function in cells of the skin, specifically subcutaneous adipocytes located there. It will be appreciated that the present cosmetic products will contain a mixture of different ingredients known to the skilled person, ensuring a fast penetration of the objective substance into the skin and preventing degradation thereof during storage.

Another high important composition according to the present invention is food material, since it provides a convenient mode of having the objective substance incorporated by an individual. Consequently, the present invention provides a food composition that prevents the onset and/or development of disorders represented by or associated with obesity, e.g. diabetes, such as a composition selected from the group consisting of milk, or fermented milk products, such as e.g. yogurt, curd, cheese, milk based fermented products, ice-creams, milk based powders, infant formulae, cereal products and fermented cereal based products, mineral water, chocolate or pet food containing at least a substance capable of essentially stimulating and/or modifying CD_{1d} function. Since the objective compound will be contained in a food material in amounts, that do not affect the original taste thereof, the consumer will not notice any change in the product, but will experience the beneficial effects thereof, namely a protective or even curing effect. Once the food material has been ingested the objective substances will arrive at the target cells or target tissue, which may be adipocytes in white or brown adipose tissue, or any other cell, which expresses CD_{1d} and regulates lipid metabolism e.g. hepatocytes, adrenal cells, pancreatic cells, and will bind to the CD_{1d} receptor and exert its activity.

Since cells bearing CD_{1d} have been found in a number of tissues, such as the liver, the small intestine, the colon, the kidney, gall bladder, the prostate, adrenal gland, the uterus, the pancreas, breast, skin and conjunctivia, the choice of the composition as detailed above will, by and large depend on the target tissue. As will be understood, for skin a cosmetic product might be the composition of choice, while in case of delivering the objective substance directly to the gut or the colon, a food product may be first choice. However, a food product may also be suitable for delivering the objective substance or substances to other organs, such as the adipose tissue, gall bladder, pancreas, the kidney or the liver, which will depend on the stability of the substance in the body and its capacity of being absorbed by the body in the gut. Since food is a daily ingested material such a product offers a great variety of different possibilities. Yet, in case the objective substance is prone to degradation in the gut a pharmaceutical composition may be selected, providing e.g. encapsulation or other galenic forms to deliver the objective substance to the target tissue/to target cells.

It will be understood that the concept of the present invention may likewise be applied as an adjuvant therapy assisting in presently used medications. In this respect the pharmaceutical composition of the present invention may be administered together with known regimens for treating obesity or diabetes and the like. Since the substance(s) of the present invention may easily be administered together with food material, special clinical food may be applied containing a high amount of the objective substances.

It will be clear that on reading the present specification together with the appending claims the skilled person will envisage a variety of different alternatives to the specific embodiments mentioned herein.

In principle, the substances according to the present invention may be used for the treatment and/ or prevention of obesity and associated diseases, such as sleep apnoea, hernias, flat feet, arthritis, osteoarthritis, some type of cancers, varicose veins, gout, respiratory problems, gall bladder disease and liver disease, atherosclerosis, coronary artery disease, stroke, hypertension, and diabetes.

In order to arrive at additional substances having the above characteristics the present invention also provides a method for screening for such substances. In this method pre-adipocytes and/or adipocytes are utilized that may be present in form of a primary culture, i.e. directly derived from an individual, or in form of a cell line. For carrying out the method, a cell culture is particularly preferred, since it allows for the continuous supply of pre-adipocyte and/or adipocyte cells during the experiments. Care must be taken that the cell culture of pre-adipocyte and/or adipocyte cells used exhibit the same phenotypic traits as do cells of a primary culture or cells directly obtained from a tissue sample. It will be understood that the person skilled in the art will select the starting material depending on the assay. Hence, if a first round assay is to be carried out a cell culture design seems to be most appropriate, while in case for additional rounds, i.e. assessing the activity of potential candidates, the tissue or even the animal model seems to be more appropriate.

The pre-adipocyte and/or adipocyte cells are exposed to a substance of interest for a time period sufficient to ensure contact of the substance with the cells. In a next step, the effect of the substance of interest on adipocyte cell development is assessed, when culturing the pre-adipocyte and/or adipocytes under normal conditions or when culturing the cells with a substance or mixture of substances known to increase adipose tissue size, especially a substance which induces/increases pre-adipocyte proliferation, differentiation and/or induces/increases adipocyte volume; e.g. glucocorticoids, insulin-like growth factor (IGF-I), angiotensin II, prostacyclin, E2Fs, norepinephrine, fatty acids, prostaglandins, lipoproteins (VLDL, LDL, HDL), macrophage colony stimulating factor, using one or more of the following assays: A) adipocyte differentiation B) adipocyte dedifferentiation C) adipocyte hyperplasia D) adipocyte proliferation E) adipocyte apoptosis F) adipocyte lipogenesis G) adipocyte lipolysis H) adipocyte lipid uptake/transport I) production of adipokines (e.g IL-6, TNFα) J) production of anti-inflammatory molecules (e.g. IL-1 receptor antagonist) K) adipocyte endocrine functions (e.g. leptin, adiponectin, resistin) L) transcription factor activity (e.g PPARs, RARs, SREBPs, HNFs) M) ABC transporter activity e.g. ABC1, ABCD2, PMP70 N) adipocyte hypertrophy O) enzyme activity including lipases (e.g. secretory and cytosolic phospholipase A₂, lipoprotein lipase, lysosomal acid lipase, HMG CoA reductase) and secretion (e.g plasminogen activator inhibitor -1) P) Cox-2 activity Q) lipoprotein receptor activity/expression. It will be understood that also more than one substance may be tested at the same time, that is a cocktail of one or more substances, which might prove beneficial for the second or further round of assaying. Additionally, as will become apparent from the subsequent description, the substance(s) of interest may also be added after exposing the cells to molecules which induce/increase pre-adipocyte proliferation, differentiation and/or induce/increase adipocyte volume.

The results obtained are compared subsequently with a control, which may simply be an assay, wherein the same type of cells have not been contacted with the test substance (but otherwise treated/cultured in the same manner), wherein a decrease/inhibition of development indicators as determined above, is indicative for a substance capable of modifying and/or stimulating endogenous CD_{1d} function. Likewise, another control may be cells lacking CD_{1d} that have been exposed to the test substance (negative control) or including a substance with a known positive effect on adipocyte/pre-adipocyte development in the assay (that is reducing or preventing adipocyte development/proliferation) and determining the difference in effect achieved by the substance investigated and the known substance (positive control).

A substance that is capable to stimulate CD_{1d} activity therein has the effect that it decreases/inhibits normal adipocyte development or any effect associated with increased adipose tissue mass, such as increased adipose proliferation, differentiation and/cell volume, whilst having no effect on the negative control.

The substance and/or compositions according to the present invention, preferably a CD_{1d} cDNA or the CD_{1d} gene or part thereof may be utilized in gene therapy, in particular for treating symptoms associated with obesity.

The following examples illustrate the invention in more detail without restricting the same thereto.

### Example 1

### Generation of CD_{1d} mutant mice

Mouse CD_{1d} is encoded by two genes, CD_{1d1} and CD_{1d2}, that share a high degree of nucleotide sequence identity (Bradbury et al., EMBO J., 7 (1988), 3081-3086). The product of the CD_{1d1} gene is recognized by all anti-CD₁ antibodies that have been described, whereas surface expression of the CD_{1d2} product has not yet been demonstrated. In addition, the predicted α2 domain of the CD_{1d2} gene product lacks an intra-domain disulfide bond that is found in the α2 domain of all published classic and non-classic MHC class I molecules (Bradbury, supra). This disulphide bond is thought to be critical for the folding of the antigen-binding groove. Thus, the CD_{1d2} gene may not encode a functional antigen-presenting molecule, and all functions previously attributed to mouse CD₁ may be effected by the product of the CD_{1d1} gene. For this reason, it was decided to introduce a targeted mutation into the CD_{1d1} gene, while leaving CD_{1d2} intact.

The CD_{1d} gene was isolated from a strain 129/Sv phage library with a probe generated by polymerase chain reaction. The targeting construct was prepared using a 2.8 kb Apal fragment containing the 5' region of the CD_{1d} gene, a 3.2 kb BamHI-Notl fragment containing the 3' region of the CD_{1d} gene (the NotI site in this fragment comes from the pBluescript vector into which phage DNA was initially subcloned), a neomycin resistance gene (neo), and the pBluescript plasmid (Stratagene). This construct was designed to delete a fragment of about 200 bp from the exon encoding the α2 domain of CD1d1. The strain 129/Sv-derived embryonic stem (ES) cell line TL1 was transfected with the NotI-linearized targeting vector. G418-resistant colonies were selected and isolated as described in Van Kaer et al., Cell 71 (1992), 1205-1214. Genomic DNA from individual clones was digested with EcoRI and hybridized with a 2.3 kb CIaI-EcoRI probe from the 5' end of the CD1d1 gene. Recombination was confirmed by digestion with KpnI and hybridization with a 700 bp BamHI-EcoRI probe from the 3' end of the CD_{1d1} gene. Chimeric mice were mated with C57BL/6 mice to score for germline transmission, and heterozygous mutant mice were intercrossed to obtain (C57BL/6x129/Sv) F2 homozygous mutants. Mice were typed for their CD1d1 status by genomic southern blotting with the 5' probe. Mutant mice were healthy and bred normally.

Because the ES cells and mouse strain used to generate mutant animals differ in their TL status (129/Sv is a TL+ stain and C57BL/6 is a TL- strain) all mice used in this study were genotyped for TL. To type mice for their TL status, tail DNA was digested with BglII and hybridized with a TL-specific probe that detects a polymorphism between strains 129/Sv (TL+) and C57BL/6 (TL-) (Pontarotti et al., Proc. Natl. Acad. Sci. USA 83 (1986), 1782-1786). This probe was generated by polymerase chain reaction using a set of primers designed on the basis of published sequences (Pontarotti, supra): 5’-TATACAGAGCTCCGTAGGAC-3’ ; and

5’-AGTTGTCTGCAGCCACGAAC-3’.

The CD_{1d1} mutant and wild-type mice were housed in a specific-pathogen-free barrier animal facility, accredited by the American Association for Accreditation of Laboratory Animal Care (AAALAC). Animals were used between 12-16 weeks or 32 weeks of age at the start of the experiments. They were housed in filter-protected cages with a 12h light-dark controlled cycle, and provided with autoclaved NIH open formula mouse chow and water ad libidum.

The institutional Animal Care and Use Committee approved all procedures. Within each experiment all mice were aged- and sex-matched. It is underlined that other genetic backgrounds can be used for creating a CD_{1d} mutant mouse, such as Balb/C genetic background.

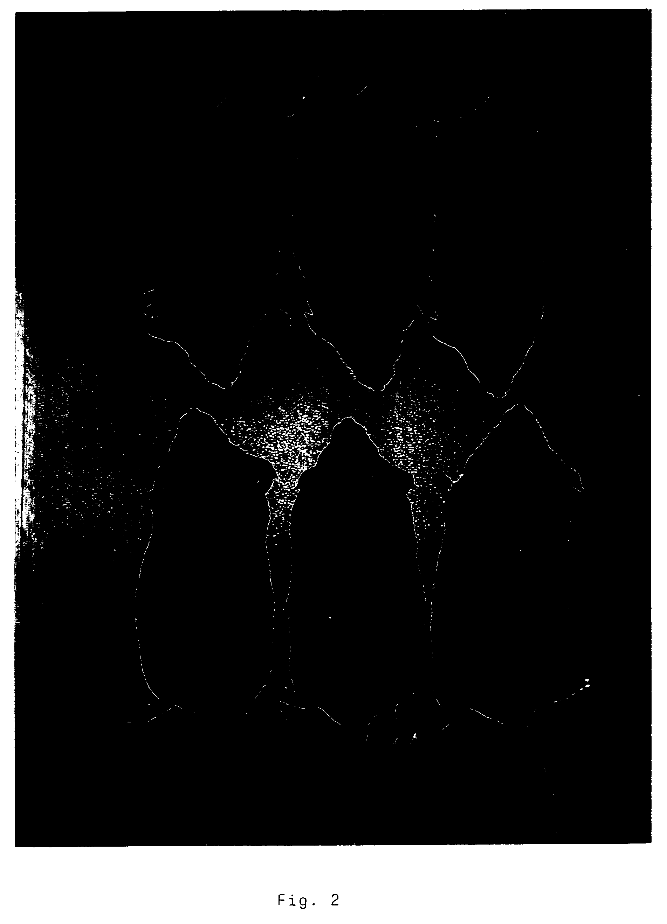

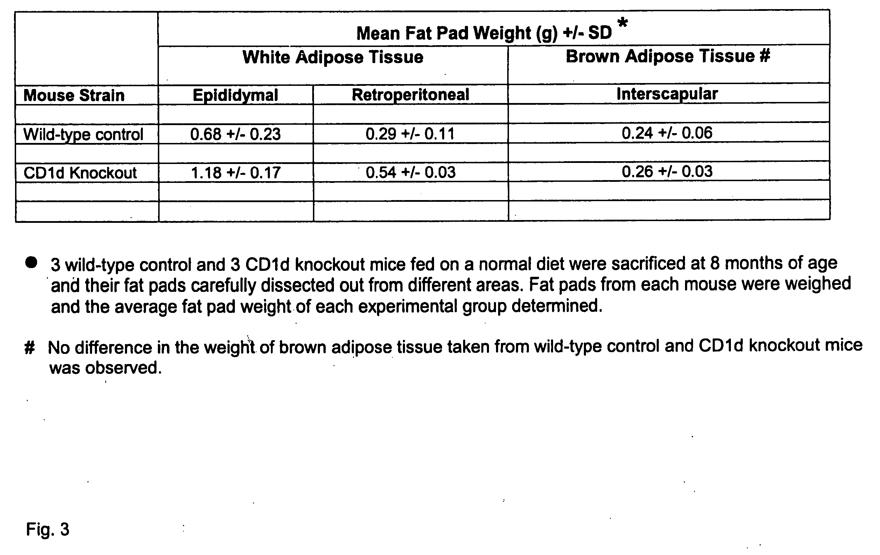

It was observed that all CD_{1d}^{-/-} mice, regardless of sex and age were on average heavier than their wild-type controls (Fig 1.). Further, at approximately 8 months of age CD1d knockout mice exhibited an obese phenotype compared to their wild-type controls (Fig.2) which is supported by their increased weight (5-6g) and the size of their fat pads (Fig.3).

### Example 2

### Gene profiling

In order to elucidate CD_{1d} function with respect to the obese phenotype of the CD_{1d}^{-/-} mice, a gene profiling assay had been performed, wherein wild-type and CD_{1d} knockout mive gene expression was compared.

Skin tissue containing the subcutaneous fat layer was extracted from 5 individual wild-type and CD_{1d} knockout mice (Age 4 months) and extracted separately using Trizol kit (Invitrogen AG, Basel, Switzerland) and then Qiagen RNeasy mini-kits (Basel Switzerland) according to manufacturer instructions with DNase I treatment to remove any genomic DNA contamination. RNA samples were quantified by OD then analyzed via dynamic gel electrophoresis with the Agilent Bioanalyser for intact 28S and 18S rRNA (All 28 / 18 ratio's were between 1.6 and 2.0). Study samples were judged to contain sufficient amounts of high-quality RNA for hybridization to GeneChips. As another quality control measure, prior to hybridization with Affymetrix GeneChips (Affymetrix, Inc., Santa Clara, CA), we confirmed that all samples gave strong signals for pre-selected genes, using Affymetrix test chips (Test chip 5' / 3' ratios were less than 3.0).

For skin, 10 µg total RNA was the starting material for all individual mouse samples. In general, total RNA was converted to biotinylated cRNA, hybridized in the Affymetrix probe array cartridge, stained, and then quantified. First and second strand cDNA synthesis was performed using the Superscript Choice System (Invitrogen AG, Basel, Switzerland), according to manufacturer instructions, but using an oligo-dT primer containing a T7 RNA polymerase binding site. Labeled cRNA was prepared with the RNA Transcript Labeling kit (Enzo Biochem Inc., NY). Biotinylated CTP and UTP were used together with unlabeled NTPs in the reaction, and unincorporated nucleotides were removed with Nucleospin columns (Macherey-Nagel, Düren, Germany).

cRNA (20 µg) was fragmented at 94 °C for 35 min in buffer containing 200 mM Tris-acetate pH 8.1, 500mM KOAc, 150 mM MgOAc. Prior to hybridization, fragmented cRNA in hybridization mix (Buffer containing 100 mM MES, 1M NaCl, 20 mM EDTA, 0.01% Tween 20, 0.5 ng/µl BSA, 0.1 ng/µl herring sperm and Affymetrix controls), was heated to 95 °C for 5 min, cooled to 45 °C and loaded onto an Affymetrix probe array cartridge. The probe array was incubated for 16 h at 45 °C at constant rotation (60 rpm), then exposed to Affymetrix washing and staining protocol.

This protocol included:
- one wash with non-stringent buffer (6X SSPE, 0.01% Tween 20, 0,005% antifoam)
- one wash with stringent buffer (100 mM MES, 0.1 M NaCl, 0.01 % Tween 20)
- First stain with 0.01 mg/ml streptavidin-phycoerythrin conjugate (Molecular Probes) in buffer containing 100 mM MES, 1M NaCl, 0.05% Tween 20, 4 mg/ml of BSA.
- one wash with non-stringent buffer (6X SSPE, 0.01% Tween 20, 0,005% antifoam)
- Second stain with 3 µg/ml of biotinylated anti-streptavidin + 0.2 mg/ml of IgG in buffer containing 100 mM MES, 1M NaCl, 0.05% Tween 20, 4 mg/ml of BSA.
- Third stain with 0.01 mg/ml streptavidin-phycoerythrin conjugate (Molecular Probes) in buffer containing 100 mM MES, 1M NaCl, 0.05% Tween 20, 4 mg/ml of BSA.
- one wash with non-stringent buffer (6X SSPE, 0.01% Tween 20, 0,005% antifoam).

A mathematical method was developed and applied to the raw GeneChip data for the selection of differentially regulated genes. This method moves beyond setting a single fold change cut-off by considering the standard deviation (SD) in the context of absolute expression, or absolute difference intensity (ADI).

The method included the following steps: (A) data processing by the commercially available "MAS5" Affymetrix program (Santa Clara, CA, USA) and rescaling, (B) logarithmic transformation to distribution normality of the rescaled data, (C) multiple hypotheses (one per gene) analysis of variance (ANOVA) testing, (D) the determination of the robust mean within condition SD (equation 1), within bins of 200 genes ordered by mean ADI levels, to determine a significance limit SD between condition, named the REGExpress function (equation 2 from Genome Biology 2001 2(12): preprint0009.1-0009.31); and (E) subsequent ranking of genes by the p value of the REGExpress and ANOVA, to help focus at effect importance. The selection is made with the p value resulting from multiple hypotheses (one per gene) ANOVA testing and/or with the p value resulting from REGExpress.

Probe arrays were scanned at 488 nm using an Argon-ion Laser (made for Affymetrix by Agilent). Readings from the quantitative scanning were analyzed with Affymetrix Gene Expression Analysis Software.

The findings are summarized in Table 1. The fold increase (+) or decrease (-) is the statistically significant relative fold increase or decrease of a gene expressed in CD1d knockout mice compared to the same gene expressed in wild-type mice. It becomes clearly evident that in CD1d knock out mice genes known to be involved in obesity and diabetes mellitus are deregulated.

| **Genes which regulate lipid metabolism and glucose metabolism:** | | | | | | |
|---|---|---|---|---|---|---|
| Gene Name | Fold increase/decrease | Mean Wt | Mean CD_{1d}-/- | Expression | Biological Function | Disease Association |
| HMG CoA reductase | +1.601 | 4.305 | 4.776 | adipose | Cholesterol Biosynthesis | Obesity, Type II Diabetes |
| Natriuretic peptide receptor | +4.572 | 1.585 | 3.105 | adipose | Induces lipolysis in adipose tissue | Obesity, Type II Diabetes |
| Glucocorticoid receptor 1 | +1.811 | 3.789 | 4.383 | adipose | glucocorticoid binding. | Obesity, Type II Diabetes |
| ABCD2 (ALDR) (member of ABC transporter family) | +1.633 | 4.011 | 4.502 | adipose | Metabolism of very long chain fatty acids. | adreno-leukodystrophy. |
| leptin | +3.669 | 3.601 | 4.901 | adipose | | Diabetes |
| Adrenergic receptor, beta 3 | +1.472 | 4.743 | 5.130 | adipose | Lipolysis, thermogenesis. | Obesity, Type II Diabetes. |
| Growth hormone receptor | +1.455 | 5.184 | 5.560 | adipose | counteracts insulin action on lipid and glucose metabolism. | Obesity, type II Diabetes. |
| SORL1 (low density lipoprotein receptor) | +3.050 | 2.354 | 3.469 | new gene. expression in adipose unknown | lipoprotein metabolism | Obesity |
| RAMP3 (Calcitonin receptor activity modifying protein) | -0.601 | 4.465 | 3.957 | adipose | Regulates the calcitonin gene related peptide (CGRP) receptor. | Obesity |
| phoshatidyl-glycerophosphate synthase | +3.516 | 2.372 | 3.629 | epidermis | phospholipid metabolism | |
| beta-1,4-galactosyltransferase | +1.388 | 6.382 | 6.710 | epidermis, adipose | Glucose metabolism | |
| nuclear encoded mitochondrial acyltransferase | +1.596 | 3.666 | 4.134 | epidermis,adipose | Metabolism | |
| PMP70 (ABC transporter) | -0.412 | 7.101 | 6.214 | epidermis, adipose | metabolic transport of long-chain acyl-CoA across peroxisomal membranes. | |
| Plasminogen activator inhibitor (PAI-1) | -0.357 | 3.906 | 2.875 | epidermis, adipose | serine protease inhibitor | Obesity, Type II Diabetes |
| MRP14 | -0.202 | 3.943 | 2.344 | dermis | A fatty acid binding protein, specifically poly unsaturated fatty acids. | |
| RAB4A | - 0.680 | 4.933 | 4.547 | epidermis, adipose | vesicular transport (early endosomes and recycling vesicles). Involved in insulin regulated translocation of the glucose transporter GLUT4. | |
| Interleukin-6 | - 0.268 | 1.882 | 0.565 | epidermis, adipose | | Obesity |

## Claims

1. A substance for treating and/or preventing obesity in an individual **characterized in that** it is capable to modify and/or stimulate endogenous CD_{1d} function, and **in that** it is obtainable by a process comprising the steps of :
(a) exposing pre-adipocytes and/or adipocytes to a substance of interest,
(b) determining the effect of the substance of interest to decrease/inhibit normal adipocyte/pre-adipocyte cell development, or decrease/inhibit the ability of a molecule to induce increased adipocyte development by screening for one or more of the following assays,
(i) adipocyte hyperplasia,
(ii) adipocyte proliferation,
(iii) adipocyte apoptosis,
(iv) adipocyte differentiation,
(v) adipocyte dedifferentiation,
(vii) production of adipokines,
(viii) production of anti-inflammatory molecules,
(ix) adipocyte lipogenesis,
(x) adipocyte lipolysis,
(xi) adipocyte lipid uptake/transport,
(xii) adipocyte endocrine functions,
(xiii) adipocyte transcription factor activity,
(xiv) ABC transporter activity,
(xv) Cox-2 activity,
(xvi) lipoprotein receptor activity/expression,
(xvii) adipocyte enzyme activity and/or secretion,
(c) comparing the results obtained with a control,
(d) wherein a decrease/inhibition of development indicators as determined in b) is indicative for a substance capable of modifying and/or stimulating endogenous CD_{1d} function.

2. The substance according to any of the preceding claims, which is a compound increasing the transcription and/or translation of the CD_{1d} gene.

3. The substance according to claim 2, which is a polynucleotide sense to a sequence comprised by the CD_{1d}-gene and/or the CD_{1d}-mRNA.

4. The substance according to claim 2, which is the CD_{1d}-gene and/or the CD_{1d}-mRNA.

5. The substance according to any of the claims 1 to 2, which is a polynucleotide sense to a sequence comprised by the glucosylceramide synthase gene and/or the glucosylceramide synthase mRNA.

6. The substance according to any of the claims 1 to 2, which is a polynucleotide sense to a sequence comprised by the sphingomyelinase or ceramide synthase gene and/or the sphingomyelinase or ceramide synthase mRNA.

7. The substance according to any of the claims 1 to 2, which is a polypeptide, binding to CD_{1d} and essentially stimulating or modifying CD_{1d} function.

8. The substance according to claim 7, wherein the polypeptide is an antibody or the variable part of an antibody.

9. The substance according to claims 1 to 2, which is a lipid.

10. The substance according to claim 9, wherein the lipid is a sphingolipid, especially sphingosine-1-phosphate, glycosphingolipid, phospholipid, ganglioside, sterol, fatty acid, glyceride, glycerol, phosphatidylinositol phosphate.

11. The substance according to claims 1 to 2 which is a phenol or polyphenol, particularly an epigallocatechin-3-gallate like molecule-natural or synthetic.

12. The substance according to claims 1 to 2 which is derived from Ginkgo.

13. The substance according to claims 9, 10, 11 and 12 which is derived from plants, microbes or animals.

14. A substance according to any of the preceding claims for the preparation of a carrier for the prevention and/or treatment of obesity and associated disorders.

15. A composition, containing at least a substance according to any of the preceding claims.

16. A composition according to claim 15, which is a food composition, a cosmetic composition or a pharmaceutical composition.

17. The composition according to claim 16, which is milk, yogurt, curd, cheese, fermented milks, milk based fermented products, ice-creams, milk based powders, infant formulae, cereal products, fermented cereal based products, mineral water, chocolate or pet food, or lotions, creams, and/or ointments or tablets, liquid, dried oral supplement, wet oral supplement, dry tube-feeding or wet tube-feeding or an anti-cancer drug.

18. Use of a substance according to any of the claims 1 to 14 or a composition according to any of the claims 15 to 17 for the preparation of a carrier for the prevention and/or treatment of obesity and associated disorders.

19. The use according to claim 18, wherein the disorder is sleep apnoea, hernias, flat feet, arthritis, osteoarthritis, hypertension, some type of cancers, varicose veins, gout, respiratory problems, gall bladder disease and liver disease, coronary artery disease, atherosclerosis, stroke, and particularly diabetes.

20. A method for identifying CD_{1d} stimulating or modifying substances, which comprises the following steps:
(a) exposing pre-adipocytes and/or adipocytes to a substance of interest, and
(b) optionally exposing the pre-adipocytes and/or adipocytes of step a) to a molecule which increases/enhances adipocyte cell development,
(c) determining the effect of the substance of interest to its capability to either decrease/inhibit normal adipocyte development, or to decrease/inhibit the ability of a molecule that induces increased adipocyte cell development, by screening for one or more of the following assays,
(i) adipocyte hyperplasia,
(ii) adipocyte proliferation,
(iii) adipocyte apoptosis,
(iv) adipocyte differentiation,
(v) adipocyte dedifferentiation,
(vi) adipocyte hypertrophy,
(vii) production of adipokines,
(viii) production of anti-inflammatory molecules,
(ix) adipocyte lipogenesis,
(x) adipocyte lipolysis,
(xi) adipocyte lipid uptake/transport,
(xii) adipocyte endocrine functions,
(xiii) adipocyte transcription factor activity,
(xiv) ABC transporter activity,
(xv) Cox-2 activity,
(xvi) lipoprotein receptor activity/expression,
(xvii) adipocyte enzyme activity and/or secretion
(d) comparing the results obtained with a control,
wherein a decrease/inhibition of development indicators as determined in c) is indicative for a substance capable of modifying and/or stimulating endogenous CD_{1d} function.

21. The method according to claim 20, wherein the substance used to induce increased adipocyte cell number and/volume is a hormone, neuropeptide, lipid especially a fatty acid, eicosanoid, adipokine, insulin -like growth factor, E2F, and/or lipoprotein.

22. The method according to claim 20, wherein the adipokines include IL-6 and TNFα.

23. The method according to any of the claim 20, wherein the anti-inflammatory molecule includes IL-1 receptor antagonist.

24. The method according to any of the claim 20, wherein the lipids are selected from the group consisting of fatty acids, phospholipids, sphingolipids, gangliosides and glycosphingolipids.

25. The method according to any of the claim 20, wherein the ABC transporters are the ABC1, ABCB, ABCC, ABCD, ABCE, ABCF and ABCG subfamilies.

26. The method according to any of the claim 20, wherein the transcription factors include PPARs, RARs, SREBPs, HNFs, and NFkappaB.

27. The method according to any of the claim 20, wherein the enzymes include secretory and cytosolic phospholipase A₂, lipoprotein lipase, lysosomal acid lipase, HMG CoA reductase and plasminogen activator inhibitor-1.

28. The method according to any of the claim 20, wherein the lipoprotein receptors are LDL, HDL and VLDL.

29. The method according to any of the claim 20, wherein the adipocte endocrines include leptin, adiponectin, and resistin.

30. Use of cells expressing and/or over-expressing CD_{1d} in an assay for screening for substances modifying and/or stimulating CD_{1d}.

31. Use of a gene reporter system for screening for substances stimulating the CD_{1d} promoter activity.

32. Use of CD_{1d}^{-/-} animals as a test model for determining the activity of substances influencing obesity and associated disorders.

33. Use of a substance according to any of the claims 1 to 14 in gene therapy.

34. The use according to claim 33, wherein the substance is a CD_{1d} cDNA.

35. Use of an assay for determining CD_{1d} gene polymorphism linked to overweight/obesity treatment.
